# EUROPEAN PATENT APPLICATION

(11) **EP 3 095 445 A1**
(43) Date of publication of application: **23.11.2016**
(21) Application number: 16159431.2
(22) Date of filing: 12.10.2010
(51) Int. Cl.: A61K 31/421, A61K 31/4192, A61P 31/04, A61P 31/00, A61K 47/12

(54) **PHARMACEUTICAL COMPOSITIONS COMPRISING RADEZOLID**

(30) Priority: 13.10.2009 US 251023 P
(62) Divisional of application: 10823926.0
(71) Applicant: Melinta Therapeutics, Inc., New Haven, CT 06511 (US)
(72) Inventor: LI, Danping, Middlebury, CT 06762 (US); BURAK, Eric S., East Haddam, CT 06423 (US)
(74) Representative: Harris, Jennifer Lucy

(57) **Abstract**

The present invention relates to pharmaceutical compositions useful for administration for treating, preventing, or reducing the risk of microbial infections.

## Description

### RELATED APPLICATION

The present application claims priority to U.S. provisional application no. 61/251,023, filed October 13,2009.

### FIELD OF THE INVENTION

The present invention relates to pharmaceutical compositions useful for administration for treating, preventing, or reducing the risk of microbial infections.

### BACKGROUND

An appropriate pharmaceutical carrier system is generally a requirement for the safe and effective delivery of a pharmaceutical active. The entire pharmaceutical composition, i.e. the pharmaceutical drug active formulated in a pharmaceutical carrier, can affect the bioavailability and also the pharmacokinetics and pharmacodynamics of the active. It is therefore important that a pharmaceutical composition be carefully developed and manufactured to deliver the desired pharmaceutical active in a safe and effective manner.

The delivery of antimicrobial agents for treating microbial infections can present special challenges. To provide therapeutic efficacy, it is generally desired that the antimicrobial agent be administered to the patient to achieve systemic concentrations in the bloodstream or target organs above a minimum inhibitory concentration (i.e. the MIC) and for a sufficient time against the particular microbial organism or organisms being targeted. Consequently, an antimicrobial agent that otherwise exhibits an effective antimicrobial profile *in vitro* can be ineffective, or even harmful, unless properly formulated for *in vivo* administration.

Therefore, the development and manufacture of suitable pharmaceutical compositions for the safe and effective delivery of pharmaceutical drug actives, in particular of antimicrobial agents, are important and ongoing needs. The present invention will be seen to meet these and other needs.

### SUMMARY OF THE INVENTION

The present invention relates to pharmaceutical compositions useful for administration for treating, preventing, or reducing the risk of microbial infections in a patient. The present invention also relates to methods for making these pharmaceutical compositions and to the use of a pharmaceutical composition in the preparation of a medicament for treating, preventing, or reducing the risk of microbial infections in a patient.

The present invention relates to a pharmaceutical composition comprising;
(a) an oxazolidinone antimicrobial agent or a pharmaceutically acceptable salt, ester, or prodrug thereof,
(b) a buffer,
(c) a pH modifier, and
(d) a solvent.

In other embodiments the present invention relates to a composition wherein said oxazolidinone antimicrobial agent comprises a pharmaceutically acceptable amount.

In other embodiments the present invention relates to a composition wherein said oxazolidinone antimicrobial agent comprises a prophylactically effective amount.

In other embodiments the present invention relates to a composition wherein said oxazolidinone antimicrobial agent is radezolid, linezolid, torezolid, or a pharmaceutically acceptable salt or prodrug thereof.

In other embodiments the present invention relates to a composition wherein said oxazolidinone antimicrobial agent is radezolid or a pharmaceutically acceptable salt thereof.

In other embodiments the present invention relates to a composition wherein said pharmaceutically acceptable salt is a hydrochloride salt.

In other embodiments the present invention relates to a composition wherein said oxazolidinone antimicrobial agent is radezolid monohydrochloride.

In other embodiments the present invention relates to a pharmaceutical composition wherein said buffer comprises citric acid and a citric acid salt.

In other embodiments the present invention relates to a pharmaceutical composition wherein said buffer comprises citric acid and sodium citrate tribasic.

In other embodiments the present invention relates to a pharmaceutical composition wherein said pH modifier is selected from sodium hydroxide and phosphoric acid.

In other embodiments the present invention relates to a pharmaceutical composition wherein said pH modifier is sodium hydroxide.

In other embodiments the present invention relates to a pharmaceutical composition wherein said pH modifier is phosphoric acid.

In other embodiments the present invention relates to a pharmaceutical composition wherein said sugar is dextrose.

In other embodiments the present invention relates to a pharmaceutical composition wherein said solvent is water.

In other embodiments the present invention relates to a pharmaceutical composition comprising

| **Ingredient** | **Mg/mL** |
|---|---|
| Radezolid hydrochloride (amount as hydrochloride salt) | 3.009 |
| Citric acid anhydrous | 4.00 |
| Sodium citrate tribasic (dihydrate) | 1.22 |
| 5% Dextrose solution (D5W) | q.s. volume |
| 1 Normal Sodium hydroxide or phosphorphic acid | q.s. pH |
| Total, mg | 1007 |
| Final pH | 3.5 (±0.1) |

| | |
|---|---|
| Active Pharmaceutical Ingredient (API) supplied as monohydrochloride salt, 3.009 mg salt = 2.778 mg as free base | |

In other embodiments the present invention relates to a pharmaceutical composition comprising

| **Ingredient** | **% (weight/weight)** |
|---|---|
| Radezolid hydrochloride (amount as hydrochloride salt) | 0.30% |
| Citric acid anhydrous | 0.40% |
| Sodium citrate tribasic (dihydrate) | 0.12% |

| 5% Dextrose solution (D5W) | q.s. volume |
|---|---|
| 1 Normal Sodium hydroxide or phosphorphic acid | q.s.pH |
| Total, mg | 100.00% |
| Final pH | 3.5 (±0.1) |

In other embodiments the present invention relates to a pharmaceutical composition for intravenous adminsitration.

In other embodiments the present invention relates to a pharmaceutical composition for injectable adminsitration.

In other embodiments the present invention relates to a method of treating a microbial infection in a patient comprising administering a pharmaceutically effective amount of a pharmaceutical composition.

In other embodiments the present invention relates to a method of preventing a microbial infection in a patient comprising administering a prophylactically effective amount of a pharmaceutical composition.

In other embodiments the present invention relates to a method of reducing the risk of a microbial infection in a patient comprising administering a prophylactically effective amount of a pharmaceutical composition.

In other embodiments the present invention relates to a pharmaceutical composition for treating a microbial infection in a patient.

In other embodiments the present invention relates to a pharmaceutical composition for preventing a microbial infection in a patient.

In other embodiments the present invention relates to a pharmaceutical composition for reducing the risk of a microbial infection in a patient.

In other embodiments the present invention relates to the use of an antibiotic compound in the manufacture of a pharmaceutical composition for treating a microbial infection in a patient comprising administering a pharmaceutically effective amount of said pharmaceutical composition to said patient.

In other embodiments the present invention relates to the use of an antibiotic compound in the manufacture of a pharmaceutical composition for preventing a microbial infection in a patient comprising administering a prophylactically effective amount of said pharmaceutical composition to said patient.

In other embodiments the present invention relates to the use of an antibiotic compound in the manufacture of a pharmaceutical composition for reducing the risk of a microbial infection in a patient comprising administering a prophylactically effective amount of said pharmaceutical composition to said patient.

In other embodiments the present invention relates to a method, composition, or use wherein said patient is a human or an animal.

In other embodiments the present invention relates to a method, composition, or use wherein said patient is a human.

The foregoing and other aspects and embodiments of the present invention can be more fully understood by reference to the following detailed description and claims.

### DETAILED DESCRIPTION OF THE INVENTION

The present invention relates to pharmaceutical compositions useful for administration to a patient for treating, preventing, or reducing the risk of microbial infections. These compositions comprise an oxazolidinone antimicrobial agent, a buffer, a pH modifier, and a solvent.

### 1. Definitions

The terms "carrier" or "carrier system" means one or more compatible substances that are suitable for delivering, containing, or "carrying" a pharmaceutical active ingredient for administration to a patient or subject.

The terms "patient" or "subject", as used herein, mean a human or an animal. Examples of animals include domesticated animals, nonlimiting examples of which include household companion animals such as cats and dogs, food animals such as cattle, sheep, goats, pigs, poultry, fish, and shellfish, zoo and other exhibition animals, and work and other animals such horses, llamas, rabbits, etc.

As used herein, the term "effective amount" refers to an amount of a pharmaceutical active compound, or a combination of compounds, for example an antimicrobial agent or agents, when administered alone or in combination, to treat, prevent, or reduce the risk of a disease state or condition, for example a microbial infection. The term also refers to an amount of a pharmaceutical composition containing an active compound or combination of compounds. For example, an effective amount refers to an amount of the compound present in a formulation given to a recipient patient or subject sufficient to elicit biological activity, for example, anti-infective activity, such as e.g., anti-microbial activity or anti-bacterial activity.

As used herein, the phrase "pharmaceutically acceptable" refers to those active compounds, materials, compositions, carriers, and/or dosage forms which are, within the scope of sound medical judgment, suitable for use in contact with the tissues of human beings and animals without excessive toxicity, irritation, allergic response, or other problems or complications, commensurate with a reasonable benefit/risk ratio.

As used herein, the term "pharmaceutically effective amount" refers to an amount of a pharmaceutical active compound, or a combination of compounds, for example an antimicrobial agent or agents, when administered alone or in combination, to treat, prevent, or reduce the risk of a disease state or condition, for example a microbial infection. The term also refers to an amount of a pharmaceutical composition containing an active compound or combination of compounds. For example, a pharmaceutically effective amount refers to an amount of the pharmaceutical active present in a pharmaceutical composition or formulation of the present invention or on a medical device containing a composition or formulation of the present invention given to a recipient patient or subject sufficient to elicit biological activity, for example, activity against a microbial infection.

The term "prophylactically effective amount" means an effective amount of a pharmaceutical active compound, or a combination of compounds, for example an antimicrobial agent or agents, when administered alone or in combination, to prevent, or reduce the risk of a disease state or condition, for example a microbial infection -- in other words, an amount to give a preventative or prophylactic effect. The term also refers to an amount of a pharmaceutical composition containing an active compound or combination of compounds.

The term "treating", as used herein, means to cure an already present disease state or condition, e.g. a microbial infection in a patient or subject. Treating can also include inhibiting, i.e. arresting the development of a disease state or condition, e.g. a microbial infection, and relieving or ameliorating, i.e. causing regression of the disease state or condition, e.g. a microbial infection.

The term "preventing", as used herein means, to completely or almost completely stop a disease state or condition, e.g. a microbial infection, from occurring in a patient or subject, especially when the patient or subject is predisposed to such or at risk of contracting a disease state or condition, e.g., a microbial infection. Preventing can also include inhibiting, i.e. arresting the development, of a disease state or condition, e.g., a microbial infection.

The term "reducing the risk of", as used herein, means to lower the likelihood or probability of a disease state or condition, e.g., a microbial infection, from occurring in a patient or subject, especially when the patient or subject is predisposed to such or at risk of contracting a disease state or condition, e.g., a microbial infection.

One or ordinary skill in the art will appreciate that there can be some overlap in the definitions of "treating", "preventing", and "reducing the risk of".

As used herein, "pharmaceutically acceptable salts" refer to derivatives of the pharmaceutical active compounds wherein the parent compound is modified by making acid or base salts thereof. Examples of pharmaceutically acceptable salts include, but are not limited to, mineral or organic acid salts of basic residues such as amines, alkali or organic salts of acidic residues such as carboxylic acids, and the like. The pharmaceutically acceptable salts include the conventional non-toxic salts or the quaternary ammonium salts of the parent compound formed, for example, from non-toxic inorganic or organic acids. For example, such conventional non-toxic salts include, but are not limited to, those derived from inorganic and organic acids selected from 2-acetoxybenzoic, 2-hydroxyethane sulfonic, acetic, ascorbic, benzene sulfonic, benzoic, bicarbonic, carbonic, citric, edetic, ethane disulfonic, ethane sulfonic, fumaric, glucoheptonic, gluconic, glutamic, glycolic, glycollyarsanilic, hexylresorcinic, hydrabamic, hydrobromic, hydrochloric, hydroiodic, hydroxymaleic, hydroxynaphthoic, isethionic, lactic, lactobionic, lauryl sulfonic, maleic, malic, mandelic, methane sulfonic, napsylic, nitric, oxalic, pamoic, pantothenic, phenylacetic, phosphoric, polygalacturonic, propionic, salicylic, stearic, subacetic, succinic, sulfamic, sulfanilic, sulfuric, tannic, tartaric, toluene sulfonic, and the commonly occurring amine acids, e.g., glycine, alanine, phenylalanine, arginine, etc.

The pharmaceutically acceptable salts of the present invention can be synthesized from a parent compound that contains a basic or acidic moiety by conventional chemical methods. Generally, such salts can be prepared by reacting the free acid or base forms of these compounds with a stoichiometric amount of the appropriate base or acid in water or in an organic solvent, or in a mixture of the two; generally, non-aqueous media like ether, ethyl acetate, ethanol, isopropanol, or acetonitrile are preferred. Lists of suitable salts are found in Remington's Pharmaceutical Sciences, 18th ed. (Mack Publishing Company, 1990) and Remington: The Science and Practice of Pharmacy, 20th Edition, Baltimore, MD: Lippincott Williams & Wilkins, 2000, which are incorporated by reference herein in their entirety. For example, salts can include, but are not limited to, the hydrochloride and acetate salts of the aliphatic amine-containing, hydroxyl amine-containing, and imine-containing compounds of the present invention.

Additionally, the compounds of the present invention, for example, the salts of the compounds, can exist in either hydrated or unhydrated (the anhydrous) form or as solvates with other solvent molecules. Nonlimiting examples of hydrates include monohydrates, dihydrates, etc. Nonlimiting examples of solvates include ethanol solvates, acetone solvates, etc.

The compounds of the present invention can also be prepared as esters, for example pharmaceutically acceptable esters. For example a carboxylic acid function group in a compound can be converted to its corresponding ester, e.g., a methyl, ethyl, or other ester. Also, an alcohol group in a compound can be converted to its corresponding ester, e.g., an acetate, propionate, or other ester.

The compounds of the present invention can also be prepared as prodrugs, for example pharmaceutically acceptable prodrugs. Since prodrugs are known to enhance numerous desirable qualities of pharmaceuticals (e.g., solubility, bioavailability, manufacturing, etc.) the compounds of the present invention can be delivered in prodrug form. Thus, the present invention is intended to cover prodrugs of the presently claimed compounds, methods of delivering the same and compositions containing the same. "Prodrugs" are intended to include any covalently bonded carriers that release an active parent drug of the present invention *in vivo* when such prodrug is administered to a mammalian subject. Prodrugs the present invention are prepared by modifying functional groups present in the compound in such a way that the modifications are cleaved, either in routine manipulation or *in vivo,* to the parent compound. Prodrugs include compounds of the present invention wherein a hydroxy, amino, or sulfhydryl group is bonded to any group that, when the prodrug of the present invention is administered to a mammalian subject, cleaves to form a free hydroxyl, free amino, or free sulfhydryl group, respectively. Examples of prodrugs include, but are not limited to, acetate, formate, and benzoate derivatives of alcohol and amine functional groups in the compounds of the present invention.

In the specification, the singular forms also include the plural, unless the context clearly dictates otherwise. Unless defined otherwise, all technical and scientific terms used herein have the same meaning as commonly understood by one of ordinary skill in the art to which this invention belongs. In the case of conflict, the present specification will control.

All percentages and ratios used herein, unless otherwise indicated, are by weight.

Throughout the description, where compositions are described as having, including, or comprising specific components, it is contemplated that compositions also consist essentially of, or consist of, the recited components. Similarly, where methods or processes are described as having, including, or comprising specific process steps, the processes also consist essentially of, or consist of, the recited processing steps. Further, it should be understood that the order of steps or order for performing certain actions is immaterial so long as the invention remains operable. Moreover, two or more steps or actions can be conducted simultaneously.

### 2. Compositions of the present invention

The invention relates to a method of treating a microbial infection in a patient comprising administering a pharmaceutically effective amount of a pharmaceutical composition. The invention relates to a method of preventing a microbial infection in a patient comprising administering a prophylactically effective amount of a pharmaceutical composition. The invention relates to a method of reducing the risk of a microbial infection in a patient comprising administering a prophylactically effective amount of a pharmaceutical composition.

The invention relates to a pharmaceutical composition for treating a microbial infection in a patient. The invention relates to a pharmaceutical composition for preventing a microbial infection in a patient. The invention relates to a pharmaceutical composition for reducing the risk of a microbial infection in a patient.

The invention relates to the use of an antibiotic compound in the manufacture of a pharmaceutical composition for treating a microbial infection in a patient comprising administering a pharmaceutically effective amount of said pharmaceutical composition to said patient. The invention relates to the use of an antibiotic compound in the manufacture of a pharmaceutical composition for preventing a microbial infection in a patient comprising administering a prophylactically effective amount of said pharmaceutical composition to said patient. The invention relates to the use of an antibiotic compound in the manufacture of a pharmaceutical composition for reducing the risk of a microbial infection in a patient comprising administering a prophylactically effective amount of said pharmaceutical composition to said patient.

The invention relates to a method, composition, or use wherein said patient is a human or an. In one embodiment, the invention relates to a method, composition, or use wherein said patient is a human.

The compositions of the present invention comprise the following essential and optional components.

### a. Oxazolidinone Antimicrobial Agent

Oxazolidinone antimicrobial agents and their pharmaceutically acceptable salts, esters, and prodrugs thereof, can be used in the methods, compositions, and uses of the present invention.

Oxazolidinone antimicrobial agents are described in U.S. Patent No. 7,456,206 B2, to Lou et al., issued November 25, 2008; U.S. Patent No. 7,148,219 B2, to Lou et al., issued December 12,2006, and its certification of correction of March 4, 2008; U.S. Patent No. 7,129,259 B2, to Chen et al., issued October 31, 2006, and its certificate of correction of March 6, 2007; U.S. Patent No. 6,969,726 B2, to Lou et al., issued November 29,2005, and its certificates of correction of February 27, 2007 and November 27, 2007; U.S. Patent No. 5,688,792, to Barbachyn et al., issued November 18, 1997; PCT Publication WO 2001/94342, to Dong A Pharm. Co. Ltd, published December 13,2001; and PCT Publication WO 2005/058886 to Dong A Pharm. Co. Ltd, published June 30, 2005.

Nonlimiting examples of oxazolidione antimicrobial agents useful herein include the following compound: 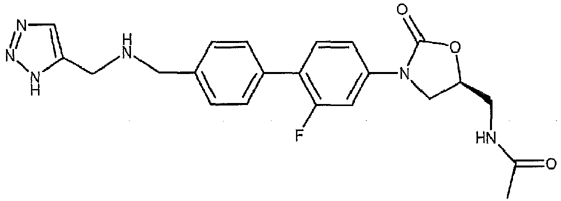 or a pharmaceutically acceptable salt or prodrug thereof. Examples of salts include the hydrochlorid salt. A further example of a salt is the monohydrochloride salt. The foregoing compound corresponds to the chemical name, inter alia, N-[3-(2-Fluoro-4'-{[(3H-[1,2,3]triazol-4-ylmethyl)-amino]-methyl}-biphenyl-4-yl)-2-oxo-oxazolidin-5-(S)-ylmethyl]-acetamide. This compound is also known by the USAN, radezolid, and corresponds to the CAS registry number 869884-78-6. The monohydrochloride salt is known by the USAN, radezolid hydrochloride, and to the CAS registry number 869884-77-5.

Other oxazolidione antimicrobial agents useful herein include linezolid and torezolid.

The dose of the oxazolidinone antimicrobial agent and mode of administration of the pharmaceutical composition will depend upon the intended patient or subject and the targeted microorganism, e.g., the target bacterial organism.

The oxazolidinone antimicrobial agent is used in a weight percentage in the composition to provide the desired pharmacological properties, such as e.g. drug bioavailability from the final composition. Weight percentages of the oxazolidinone antimicrobial agent range from about 0.01% to about 5%. In further embodiments, weight percentages range from about 0.1% to about 0.5%. In further embodiments, weight percentages range from about 0.25% to about 0.40%.

### b. Buffer

The compositions of the present invention comprise a buffer. In one embodiment the buffer comprises the combination of citric acid and a citric acid salt. In a further embodiment the buffer comprisesis the combination of citric acid and sodium citrate tribasic. In a further embodiment the buffer comprisesis the combination of citric acid and sodium citrate tribasic (dihydrate).

### c. pH modifier

The compositions of the present invention comprise a pH modifier. In one embodiment the pH modifier is selected from sodium hydroxide and phosphoric acid. In another embodiment the pH modifier is sodium hydroxide. In another embodiment the pH modifier is phosphoric acid.

### d. Sugar

The compositions of the present invetnion comprise a sugar. In one embodiment the sugar is dextrose, which is also known as glucose.

### e. Solvent

The compositions of the present invention comprise a solvent. In one embodiment water is a component of the compositions of the present invention and can be used at appropriate levels to provide the desired final formulation.

### f. Other Additional Components

The compositions of the present invention can further comprise one or more additional components selected from a wide variety of excipients known in the pharmaceutical formulation art. According to the desired properties of the tablet or capsule, any number of ingredients can be selected, alone or in combination, based upon their known uses in preparing the compositions of the present invention. Such ingredients include, but are not limited to, water; nonaqueous solvents (e.g. ethanol); coatings; capsule shells; colorants; waxes, gelatin; flavorings; preservatives (e.g., methyl paraben, sodium benzoate, and potassium benzoate); antioxidants [e.g., butylated hydroxyanisole ("BHA"), butylated hydroxytoluene ("BHT"), and vitamin E and vitamin E esters such as tocopherol acetate]; flavor enhancers; sweeteners (e.g., aspartame and saccharin); compression aids; surfactants, etc.

### 3. Methods of making the pharmaceutical carriers and pharmaceuticals compositions

Useful carriers and compositions for administration can be prepared by any of the methods well known in the pharmaceutical art, described, for example, in Eds. R. C. Rowe, et al., Handbook of Pharmaceutical Excipients, Fifth Edition, Pharmaceutical Press (2006), Remington's Pharmaceutical Sciences, 18th ed. (Mack Publishing Company, 1990), Remington: The Science and Practice of Pharmacy, 20th Edition, Baltimore, MD: Lippincott Williams & Wilkins, 2000, and L. Lachman, H.A. Lieberman, J.L. Kanig (1986). The Theory and Practice of Industrial Pharmacy (3rd Ed.). Lea & Febiger, Philadelphia, which are incorporated by reference herein in their entirety.

### 4. Methods of Treating, Preventing or Reducing the Risk of Microbial Infections

The present invention also provides a method of treating, preventing, or reducing the risk of a microbial infection in a patient or subject. These methods comprise administering a pharmaceutically or prophylactically effective amount of the pharmaceutical actives of the present invention as a pharmaceutical composition or formulation from the carriers of the present invention to a patient or subject at an appropriate dosage.

One of ordinary skill in the art can select an appropriate dosage of the pharmaceutical active. In practicing the methods of the present invention, it is desired that the blood and or tissue level in the patient or subject of the compound be of an appropriate level for a sufficient time interval. As mentioned above, to provide therapeutic efficacy, it is generally desired that the antimicrobial agent be administered to the patient to achieve systemic concentrations in the bloodstream or target organs above a minimum inhibitory concentration (i.e. the MIC) and for a sufficient time against the particular microbial organism or organisms being targeted.

The pharmaceutical compositions of the present invention are useful for treating, preventing, or reducing the risk of a disorder such as a microbial infection in a patient or subject, e.g., a human, or a nonhuman mammal or other animal. This comprises the step or steps of administering a pharmaceutically effective or prophylactically effective amount of a composition of the present invention. Microbial infections or treatments include, *inter alia,* those selected from the group consisting of a skin infection, pneumonia (both nosocomial and community acquired pneumonia), post-viral pneumonia, an abdominal infection, a urinary tract infection, bacteremia, septicemia, endocarditis, an atrio-ventricular shunt infection, a vascular access infection, meningitis, surgical prophylaxis, a peritoneal infection, a bone infection, a joint infection, a methicillin-resistant *Staphylococcus aureus* infection, a vancomycin-resistant *Enterococci* infection, a linezolid-resistant organism infection, and tuberculosis.

In conjunction with the methods of the present invention, pharmacogenomics (i.e., the study of the relationship between an individual's genotype and that individual's response to a foreign compound or drug) can be considered. Differences in metabolism of therapeutics can lead to severe toxicity or therapeutic failure by altering the relation between dose and blood concentration of the pharmacologically active drug. Thus, a physician or clinician can consider applying knowledge obtained in relevant pharmacogenomics studies in determining whether to administer a drug as well as tailoring the dosage and/or therapeutic regimen of treatment with the drug.

Generally, an effective amount of dosage of the pharmaceutical active will be in the range of from about 0.1 to about 100 mg/kg of body weight/day, more preferably from about 1.0 to about 50 mg/kg of body weight/day. The amount administered will also likely depend on such variables as the disease or condition that one is intending to treat, prevent, or reduce the risk of, the overall health status of the patient, the relative biological efficacy of the parent compound delivered from the hydrogen sulfate salt, the formulation, the presence and types of excipients in the formulation, and the route of administration. Also, it is to be understood that the initial dosage administered can be increased beyond the above upper level in order to rapidly achieve the desired blood-level or tissue level, or the initial dosage can be smaller than the optimum.

### 5. Examples

### Example 1. Composition of radezolid hydrochloride intravenous formulation: 2.778 mg/ml as free base

| | | Batch size, mL | 1,000 gram batch |
|---|---|---|---|
| **Ingredient** | **Mg/mL** | **% (w/w)** | **Amt (g) / batch** |
| Radezolid hydrochloride (amount as hydrochloride salt) | 3.009 | 0.30% | 3.009 |
| Citric acid anhydrous | 4.00 | 0.40% | 4.000 |
| Sodium citrate tribasic (dihydrate) | 1.22 | 0.12% | 1.220 |
| 5% dextrose solution (D5W) | q.s. volume | q.s. | q.s. |
| 1 Normal Sodium hydroxide or phosphorphic acid | q.s.pH | q.s. | q.s. |
| Total, mg | 1007 | 100.00% | 1007.000 |
| Final pH | 3.5 (±0.1) | | |
| Density of the solution is 1.007 g/mL | | | |

| Active Pharmaceutical Ingredient supplied as monohydrochloride salt, 3.009 mg salt = 2.778 mg as free base | | | |
|---|---|---|---|
| Citric buffer as mM | 25.0 | | |

### Procedure.

1. Weigh dextrose 5% solution for Injection approximately 80% of the total batch weight into a suitable container.
2. Add the required amount of citric acid and sodium citrate tribasic (dihydrate) to the solution and mix until dissolved. Record pH.
3. Heat up the solution to 35-43°C
4. With continueous heating at 35-43°C, add radezolid HCl salt corrected for purity and mix until dissolved.
5. Lower the solution temperature to 25°C (±3)
6. Test for pH. The target pH is 3.5 (±0.1). Adjust with 1 Normal Sodium Hydroxide or Phosphoric acid as needed.
7. Q.S. to the final weight with dextrose 5% solution for Injection.
8. Test for pH. Adjust if outside of 3.5 (±0.1). Record final pH and solution weight.
9. Filter solution (0.22 um) and fill into vials.

### Example 2. Composition of radezolid hydrochloride intravenous formulation: 2.778 mg/ml as free base in 10 mM buffer

| | | Batch size, mL | 1,000 gram batch |
|---|---|---|---|
| **Ingredient** | **Mg/mL** | **% (w/w)** | **Amt (g) / batch** |
| Radezolid hydrochloride (amount as hydrochloride salt) | 3.009 | 0.30% | 3.009 |
| Citric acid (anhydrous, Mw = 192.12) | 1.50 | 0.15% | 1.500 |
| Sodium citrate tribasic (dihydrate) Mw = 294.10 | 0.65 | 0.06% | 0.650 |
| Dextrose Powder | 50 | 4.97% | q.s. |
| 1N Sodium hydroxide and/or phosphorphic acid | q.s. | q.s. | q.s. |
| Total, mg | 1007 | 100.00% | 1007.000 |
| Final pH | 3.5 | | |
| | (±0.1) | | |
| Density of the solution is 1.007 g/mL | | | |
| Active Pharmaceutical Ingredient supplied as HCl salt, 3.009 RX-1741-HCl salt = 2.778 mg as free base | | | |
| Citric buffer as mM | 10.0 | | |

### Procedure.

1. Weigh Dextrose Powder for Injection into a suitable container.
2. Add the required amount of citric acid and sodium citrate tribasic (dihydrate) to the solution and mix until dissolved. Record pH.
3. Heat up the solution to 35-43°C
4. With continueous heating at 35-43°C, add radezolid HCl salt corrected for purity and mix until dissolved.
5. Lower the solution temperature to 25°C (±3)
6. Test for pH. The target pH is 3.5 (±0.1). Adjust with 1 Normal Sodium Hydroxide or Phosphoric acid as needed.
7. Q.S. to the final weight with dextrose 5% solution for Injection.
8. Test for pH. Adjust if outside of 3.5 (±0.1). Record final pH and solution weight.
9. Filter solution (0.22 um) and fill into vials.

### INCORPORATION BY REFERENCE

The entire disclosure of each of the patent documents, including certificates of correction, patent application documents, scientific articles, governmental reports, websites, and other references referred to herein is incorporated by reference in its entirety for all purposes.

### EQUIVALENTS

The invention can be embodied in other specific forms without departing from the spirit or essential characteristics thereof. The foregoing embodiments are therefore to be considered in all respects illustrative rather than limiting on the invention described herein. Scope of the invention is thus indicated by the appended claims rather than by the foregoing description, and all changes that come within the meaning and range of equivalency of the claims are intended to be embraced therein.

Aspects and features of the present disclosure are set out in the following numbered clauses which contain the subject matter of the claims of the parent application as originally filed.
1. A pharmaceutical composition comprising;
(a) an oxazolidinone antimicrobial agent or a pharmaceutically acceptable salt, ester, or prodrug thereof,
(b) a buffer,
(c) a pH modifier, and
(d) a solvent.
2. A composition according to clause 1 wherein said oxazolidinone antimicrobial agent comprises a pharmaceutically acceptable amount.
3. A composition according to clause 1 wherein said oxazolidinone antimicrobial agent comprises a prophylactically effective amount.
4. A composition according to any of clauses 1 to 3 wherein said oxazolidinone antimicrobial agent is radezolid, linezolid, torezolid, or a pharmaceutically acceptable salt or prodrug thereof.
5. A composition according to any of clauses 1 to 4 wherein said oxazolidinone antimicrobial agent is radezolid or a pharmaceutically acceptable salt thereof.
6. A composition according to clause 5 wherein said pharmaceutically acceptable salt is a hydrochloride salt.
7. A composition according to any of clauses 1 to 3 wherein said oxazolidinone antimicrobial agent is radezolid monohydro chloride.
8. A pharmaceutical composition according to clauses 1 to 7 wherein said buffer comprises citric acid and a citric acid salt.
9. A pharmaceutical composition according to clauses 1 to 7 wherein said buffer comprises citric acid and sodium citrate tribasic.
10. A pharmaceutical composition according to clauses 1 to 9 wherein said pH modifier is selected from sodium hydroxide and phosphoric acid.
11. A pharmaceutical composition according to clauses 1 to 10 wherein said pH modifier is sodium hydroxide.
12. A pharmaceutical composition according to clauses 1 to 10 wherein said pH modifier is phosphoric acid.
13. A pharmaceutical composition according to clauses 1 to 12 wherein said sugar is dextrose.
14. A pharmaceutical composition according to clauses 1 to 13 wherein said solvent is water.
15. A pharmaceutical composition comprising

| **Ingredient** | **Mg**/**mL** |
|---|---|
| Radezolid hydrochloride (amount as hydrochloride salt) | 3.009 |
| Citric acid anhydrous | 4.00 |
| Sodium citrate tribasic (dihydrate) | 1.22 |
| 5% Dextrose solution (D5W) | q.s. |
| 1 Normal Sodium hydroxide or phosphorphic acid | q.s. |
| Total, mg | 1007 |
| Final pH | 3.5 (±0.1) |

16. A pharmaceutical composition comprising

| **Ingredient** | **% (weight/weight)** |
|---|---|
| Radezolid hydrochloride (amount as hydrochloride salt) | 0.30% |
| Citric acid anhydrous | 0.40% |
| Sodium citrate tribasic (dihydrate) | 0.12% |
| 5% Dextrose solution (D5W) | q.s. |
| 1 Normal Sodium hydroxide or phosphorphic acid | q.s. |
| Total, mg | 100.00% |
| Final pH | 3.5 (±0.1) |

17. A pharmaceutical composition according to any of clauses 1 to 16 for intravenous administration.
18. A pharmaceutical composition according to any of clauses 1 to 16 for injectable administration.
19. A method of treating a microbial infection in a patient comprising administering a pharmaceutically effective amount of a pharmaceutical composition according to any of clauses 1 to 18.
20. A method of preventing a microbial infection in a patient comprising administering a prophylactically effective amount of a pharmaceutical composition according to any of clauses 1 to 18.
21. A method of reducing the risk of a microbial infection in a patient comprising administering a prophylactically effective amount of a pharmaceutical composition according to any of clauses 1 to 18.
22. A pharmaceutical composition for treating a microbial infection in a patient according to any of clauses 1 to 18.
23. A pharmaceutical composition for preventing a microbial infection in a patient according to any of clauses 1 to 18.
24. A pharmaceutical composition for reducing the risk of a microbial infection in a patient according to any of clauses 1 to 18.
25. The use of an antibiotic compound in the manufacture of a pharmaceutical composition according to any of clauses 1 to 18 for treating a microbial infection in a patient comprising administering a pharmaceutically effective amount of said pharmaceutical composition to said patient.
26. The use of an antibiotic compound in the manufacture of a pharmaceutical composition according to any of clauses 1 to 18 for preventing a microbial infection in a patient comprising administering a prophylactically effective amount of said pharmaceutical composition to said patient.
27. The use of an antibiotic compound in the manufacture of a pharmaceutical composition according to any of clauses 1 to 18 for reducing the risk of a microbial infection in a patient comprising administering a prophylactically effective amount of said pharmaceutical composition to said patient.
28. A method, composition, or use according to any of clauses 19 to 27 wherein said patient is a human or an animal.
29. A method, composition, or use according to any of clauses 19-27 wherein said patient is a human.

## Claims

1. A pharmaceutical composition comprising:
(a) radezolid or a pharmaceutically acceptable salt thereof,
(b) a buffer comprising citric acid and a citric acid salt,
(c) a pH modifier selected from sodium hydroxide and phosphoric acid, and
(d) water.

2. A composition according to claim 1 wherein the radezolid or a pharmaceutically acceptable salt thereof comprises a pharmaceutically effective amount or a prophylactically effective amount.

3. A composition according to claim 1 wherein said pharmaceutically acceptable salt is a hydrochloride salt of radezolid.

4. A composition according to any of claims 1 or 2 wherein the radezolid or a pharmaceutically acceptable salt thereof is radezolid monohydrochloride.

5. A pharmaceutical composition according to claims 1 to 4 wherein said buffer comprises citric acid and sodium citrate tribasic.

6. A pharmaceutical composition according to claims 1 to 5 wherein said composition further comprises dextrose.

7. A pharmaceutical composition comprising
3.009 mg/mL radezolid hydrochloride, 4.00 mg/mL citric acid, 1.22 mg/mL sodium citrate tribasic dihydrate, quantum sufficit 5% dextrose solution, and quantum sufficit 1 normal sodium hydroxide or phosphoric acid, wherein the pH of the composition is 3.5±0.1,
or
0.30 wt.% radezolid hydrochloride, 0.40 wt.% citric acid, 0.12 wt.% sodium citrate tribasic dihydrate, quantum sufficit 5% dextrose solution, and quantum sufficit 1 normal sodium hydroxide or phosphoric acid, wherein the pH of the composition is 3.5±0.1.

8. A pharmaceutical composition according to any of claims 1-6, wherein the radezolid or a pharmaceutically acceptable salt thereof in the composition ranges from about 0.01% to about 5% by weight, preferably from about 0.1 % to about 0.5% by weight, and more preferably from about 0.25% to about 0.40% by weight.

9. A pharmaceutical composition according to any of claims 1 to 8 for intravenous administration or injectable administration.

10. A pharmaceutical composition according to any of claims 1 to 9 for use in a method of (i) treating a microbial infection in a patient;
(i) preventing a microbial infection in a patient; or
(ii) reducing the risk of a microbial infection in a patient.

11. A pharmaceutical composition according to claim 10 wherein said patient is a human or an animal.

12. A pharmaceutical composition according to claim 10 wherein said patient is a human.
